# EUROPÄISCHE PATENTANMELDUNG

(11) **EP 2 816 031 A1**
(43) Veröffentlichungstag der Anmeldung: **24.12.2014**
(21) Anmeldenummer: 13172454.4
(22) Anmeldetag: 18.06.2013
(51) Int. Cl.: C07D 213/61

(54) **Verfahren zur Herstellung von 2,3-Dichlorpyridin**

(71) Anmelder: Saltigo GmbH, 51369 Leverkusen (DE)
(72) Erfinder: Wehrle Bernhard, Dr., 51373 Leverkusen (DE); Lumm, Michael, Dr., 50859 Köln (DE); Markert, Robert, Dr., 51377 Leverkusen (DE); Casser, Carl, Dr, 13585 Berlin (DE)
(74) Vertreter: Pettrich, Klaus-Günter

(57) **Zusammenfassung**

Beschrieben wird ein Verfahren zur Herstellung von 2,3-Dichlorpyridin, bei dem man Verbindungen der Formel (I) (R-N=CHCH₃) mit Chloressigsäureanhydrid oder Chloressigsäurechlorid zu einem N-Ethenylchloracetamid der Formel (II) umsetzt und dieses dann zusammen mit einem Chlorierungsreagenz und einem Vilsmeier-Reagenz zu einer 2,3-Dichlorpyridiniumchlorid-Verbindung der Formel (IV) reagieren läßt, aus der durch Erwärmung das 2,3-Dichlorpyridin freigesetzt wird.

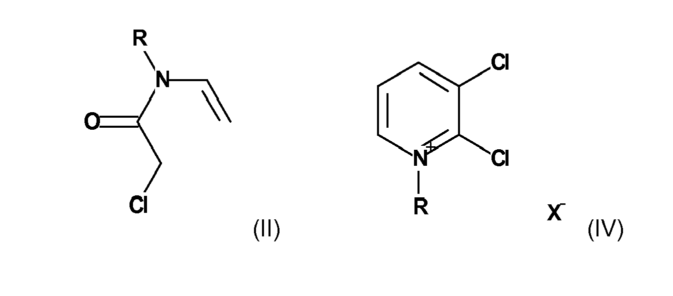

## Beschreibung

Die Erfindung betrifft ein Verfahren zur Herstellung von 2,3-Dichlorpyridin.

2,3-Dichlorpyridin ist ein wichtiger Baustein für neuartige Insektizide. Der technische Zugang zum 2,3-Dichlorpyridin erfolgt nach der WO 2005/070888 ausgehend vom Nicotinamid, welches durch Hofmann-Abbau, Chlorierung, Diazotierung und Sandmeyer-Reaktion in das 2,3-Dichlorpyridin überführt wird. Bei diese Synthesesequenz fallen große Mengen an schwermetallhaltigen Abwässern an, deren Entsorgung kostenintensiv ist und somit die Wirtschaftlichkeit des Gesamtverfahrens deutlich verringert. Es ist daher von großem Interesse, ein Verfahren zur Herstellung von 2,3-Dichlorpyridin zu finden, bei dem keine schwermetallhaltigen Abwässer anfallen.

Aufgabe der Erfindung war es daher, ein Verfahren zur Herstellung von 2,3-Dichlorpyridin zu entwickeln, bei dem leicht zugängliche Ausgangsstoffe ohne den Einsatz von Schwermetall-Katalysatoren zum gewünschten Produkt umgesetzt werden.

Überraschenderwise wurde nun ein derartiges Verfahren, gefunden, welches diese Anforderungen erfüllt.

Gegenstand der Erfindung ist daher ein Verfahren zur Herstellung von 2,3-Dichlorpyridin der Formel (VIII) 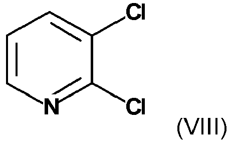 bei dem man Verbindungen der Formel (I)

R-N=CHCH₃ (I),

in der R für einen gegebenenfalls substituierten geradkettigen oder verzweigten C₁-C₈-Alkyl-, C₃-C₅-Alkenyl-, C₄-C₆-Cycloylkyl oder C₆-C₁₀- Arylrest steht,
mit Chloressigsäureanhydrid oder Chloressigsäurechlorid über die Zwischenprodukte der Formel (V) bzw. (VI) 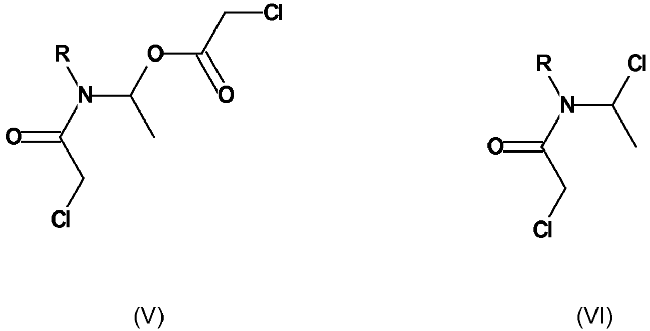 in der R die für Verbindungen der Formel (I) angegebene Bedeutung besitzt, unter Abspaltung von Chloressigsäure aus dem Zwischenprodukt der Formel (V) bzw. unter Abspaltung von Chlorwasserstoff aus dem Zwischenprodukt der Formel (VI) mittels einer Base oder einer thermischen Behandlung zu einem N-Ethenylchloracetamid der Formel (II) 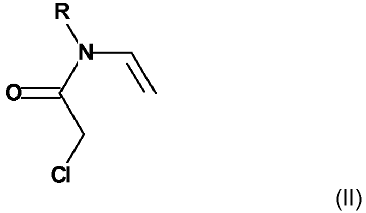 umsetzt, und diese Verbindung der Formel (II) dann zusammen mit einem Chlorierungsreagenz und einem Vilsmeier-Reagenz der Formel (III) 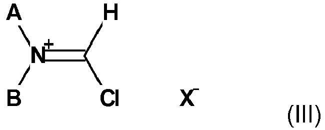 in der A und B jeweils unabhängig voneinander für einen Phenyl- oder geradkettigen oder verzweigten C₁-C₅-Alkylrest und X für ein aus dem Chlorierungsreagenz stammendes Gegenion stehen, zu einer 2,3-Dichlorpyridiniumchlorid-Verbindung der Formel (IV) 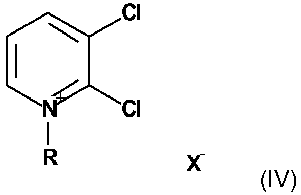 reagieren läßt, aus der durch Erwärmung das 2,3-Dichlorpyridin der Formel (VIII) freigesetzt wird.

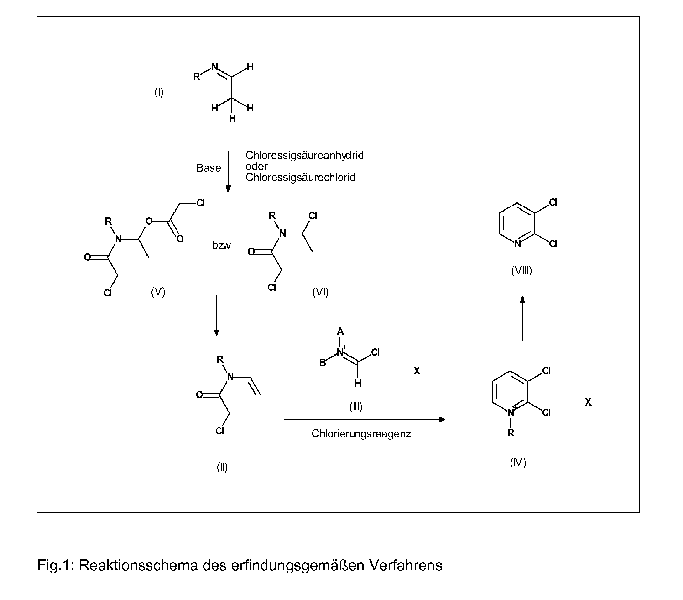

Die einzelnen Schritte des erfindungsgemäßen Verfahrens sind schematisch in Fig. 1 dargestellt.

Nach dem erfindungsgemäßenVerfahren ist es in vorteilhafter Weise möglich, 2,3-Dichlorpyridin ohne den Einsatz von Schwermetall-Katalysatoren herzustellen. Schwermetallhaltige Abwässer fallen nicht an, was das erfindungsgemäße Verfahren besonders für die großtechnische Produktion geeignet macht.

In den Formeln (I), (II), (IV),(V) und (VI) steht R für jeweils einen C₁-C₈-Alkyl, C₃-C₅-Alkenyl-, C₄-C₆-Cycloylkyl oder C₆-C₁₀- Arylrest, der gegebenfalls noch mit einen Halogen-, Halogenalkyl-, Alkoxy- oder Cyanidrest substituiert sein kann. Bevorzugt ist R Benzyl oder C₁ bis C₅-Alkyl.

In der Formel (III) steht A und B jeweils unabhängig voneinander für einen Phenyl- oder einen C₁-C₅ Alkylrest.

Üblicherweise wird das erfindungsgemäße Verfahren in einem inerten Lösungsmittel wie z.B. Hexan, Methylcyclohexan, Toluol, Xylol, Ethylbenzol, Trichlorbenzol, Chlorbenzol oder Dichlorbenzol durchgeführt.

Die Verbindungen der Formel (I) sind bekannt (J.Am.Chem.Soc.66 (1944), 82-84; J. Chem.Soc.Perkin Trans. I 1984, 1173-1182) und können aus den entsprechenden Aminen der Formel (VII),

R-NH₂ (VII)

in welcher R die oben angegebene Bedeutung hat, mit Acetaldehyd erhalten werden. Das hierbei entstehende Wasser kann durch wasserentziehende Mittel und/oder durch die Gegenwart eines mit Wasser praktisch nicht mischbaren organischen Lösungsmittels entfernt werden. Für die Erfindung sind insbesondere Amine der Formel (VII) geeignet, in welcher R für gegebenenfalls substituiertes Phenyl, Methyl, Ethyl, n- oder i-Propyl, n,- oder i-Butyl oder gegebenenfalls substituiertes Benzyl steht.

Das für die erfindungsgemäße Synthese erforderliche Vilsmeier-Reagenz der Formel (III) ist bekannt und wird aus einem Chlorierungsreagenz und einem Dialkylformamid der Formel (IX) 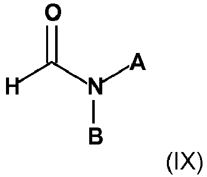 in der A und B die für Formel (III) angegebene Bedeutung besitzen, entweder in einem separaten Schritt oder in situ hergestellt. [Bull.Soc.Chim.Fr. Seite 1989 - 1999 (1962); Russ.Chem.Rev. 29, 599 (1960)]. Bevorzugte Dialkylformamide hierfür sind N,N-Dimethylformamid, N,N-Di-n-butylformamid und N-Methyl-N-phenylformamid. Bevorzugt wird das Vilsmeier-Reagenz in situ hergestellt.

Erfindungsgemäß bevorzugte Chlorierungsreagenzien sind Phosgen, Oxalylchlorid, Thionylchlorid, Phosphoroxytrichlorid. Besonders bevorzugt ist Phosgen als Chlorierungs-Reagenz. Es wird üblicherweise ein Überschuß (wie weiter unten angegeben) im Vergleich zum N-Ethenylchloracetamid der Formel (II) eingesetzt. Es dient zum einen zur Herstellung des Vilsmeier-Reagenz, z.B. in situ, und zum anderen zur Herstellung der Verbindung (IV)

X im Vilsmeier-Reagenz stammt aus dem Chlorierungsreagenz. Beim Einsatz von Phosgen oder Thionylchlorid ist X ein Chloridion, bei Phosphoroxytricchlorid ist X formal ein PO₂Cl₂-lon.

Nach dem erfindungsgemäßen Verfahren werden zur Herstellung des Ethenylchloracetamids der Formel (II) pro Mol der Verbindung der Formel (I) ca. 0,9 bis 1,3 Mol Chloressigsäureanhydrid bzw. Chloressigsäurechlorid verwendet. Die Umsetzung erfolgt bei -25°C bis 120°C in einem geeigneten Lösungsmittel wie z.B Toluol und gegebenenfalls in Gegenwart einer Base wie z.B. einem Trialkylamin wie z.B. Trimethylamin, Triethylamin, Triisopropylamin, Tripropylamin oder Tributylamin. Die Base wird in Mengen von 0,2 bis 1 Mol pro Mol der Verbindung der Formel (I) verwendet. Nach erfolgter Umsetzung werden üblicherweise die flüchtigen Anteile aus dem Reaktionsgemisch entfernt. Das verbleibende N-(1-chloracetoxyethyl)-chloracetamid der Formel (V) bzw. das N-(1-chlorethyl)-chloracetamid der Formel (VI) wird thermisch bzw. unter Einwirkung einer der weiter oben genannten Basen (Trialkylamine) unter Abspaltung von Chloressigsäure ( aus Verbindungen der Formel (V)) bzw. Chlorwasserstoff (aus Verbindungen der Formel (VI)) in das N-Ethenylchloracetamid der Formel (II) überführt.

Nach dem erfindungsgemäßen Verfahren verwendet man üblicherweise für die Herstellung der 2,3-Dichlorpyridiniumchloride der Formel (IV) pro Mol N-Ethenylchloracetamid der Formel (II) ca.1 bis 1,6 Mol Dialkylformanid und zusätzlich ca.1-5 Mol Chlorierungsreagenz. Das Verfahren wird bevorzugt in einem inerten Lösungsmittel durchgeführt, wobei die Temperatur gegebenenfalls während des Verfahrens geändert werden kann und gleichzeitig die thermische Abspaltung der Organochlorverbindung R-Cl unter Bildung des 2,3-Dichlorpyridins zwischen 60°C und 160°C erfolgt. Eine Isolierung der 2,3-Dichlorpyridiniumchlorid-Verbindung der Formel (IV) ist somit nicht erforderlich.

Eine bevorzugte Verfahrensvariante ist dadurch gekennzeichnet, dass man ein Gemisch aus Dialkylformamid und N-Ethenylchloracetamid der Formel (II) parallel mit dem Chlorierungsreagenz Phosgen zu vorgelegtem Lösungsmittel dosiert. Hierbei wird das Vilsmeier-Reagenz der Formel (III) hergestellt, indem man ein Dialkylformamid der Formel (IX) 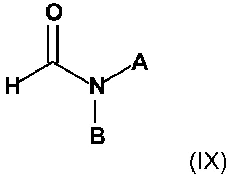 in der A und B die für Formel (III) angegebene Bedeutung besitzen, gemeinsam im Gemisch mit dem Ethenylchloracetamid der Formel (II) parallel zu dem Chlorierungsreagenz wie bevorzugt Phosgen in ein vorgelegtes Lösungsmittel eindosiert.

Es ist jedoch auch möglich, das Chlorierungsreagenz Phosgen vorab mit dem Dialkylformamid umzusetzen und in das erhaltene Reaktionsgemisch das N-Ethenylchloracetamid der Formel (II) zu dosieren. Die Entfernung des überschüssigen Chlorierungsmittels sowie des bei der Umsetzung entstandenen Dialkylamins der allgemeinen Formel ABNH, in der A und B die oben angegebene Bedeutung haben, und der Organochlorverbindung R-Cl aus dem Reaktionsgemisch erfolgen nach allgemein bekannten Verfahren und können gegebenenfalls nach Umarbeitung wieder in das Gesamtverfahren zurückgeführt werden.

## Patentansprüche

1. Verfahren zur Herstellung von 2,3-Dichlorpyridin der Formel (VIII) 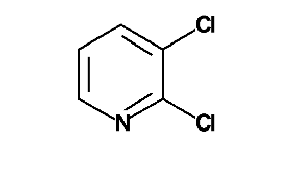 bei dem man Verbindungen der Formel (I)
R-N=CHCH₃ (I),
in der R für einen geradkettigen oder verzweigten C₁-C₈-Alkyl, C₃-C₅-Alkenyl-, C₄-C₆-Cycloylkyl oder C₆-C₁₀- Arylrest steht,
mit Chloressigsäureanhydrid oder Chloressigsäurechlorid über die Bildung der Zwischenprodukte der Formel (V) bzw. (VI) 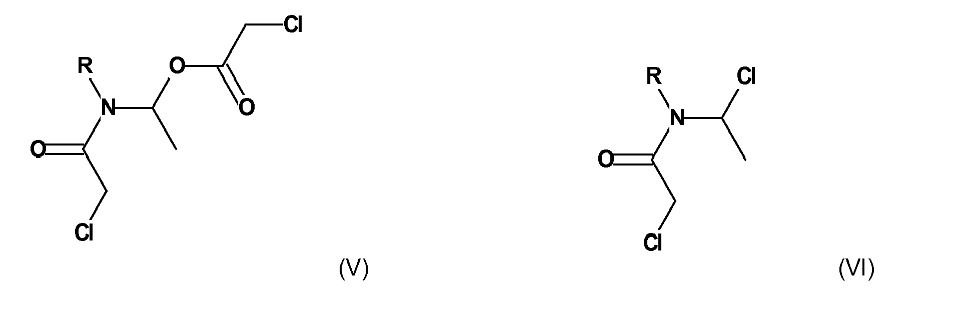 in der R die für Verbindungen der Formel (I) angegebene Bedeutung besitzt, unter Abspaltung von Chloressigsäure aus dem Zwischenprodukt der Formel (V) bzw. unter Abspaltung von Chlorwasserstoff aus dem Zwischenprodukt der Formel (VI) mittels einer Base oder einer thermischen Behandlung zu einem N-Ethenylchloracetamid der Formel (II) 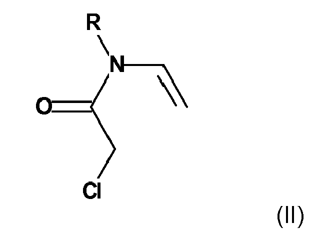 umsetzt und diese Verbindung der Formel (II) dann zusammen mit einem Chlorierungsreagenz und einem Vilsmeier-Reagenz der Formel (III) 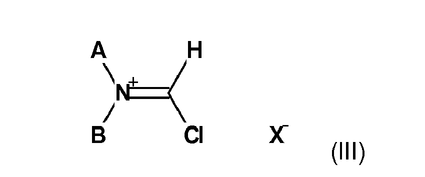 in der A und B jeweils unabhängig voneinander für einen Phenyl- oder geradkettigen oder verzweigten C₁-C₅-Alkylrest und X für ein aus dem Chlorierungsreagenz stammendes Gegenion stehen, zu einer 2,3-Dichlorpyridiniumchlorid-Verbindung der Formel (IV) 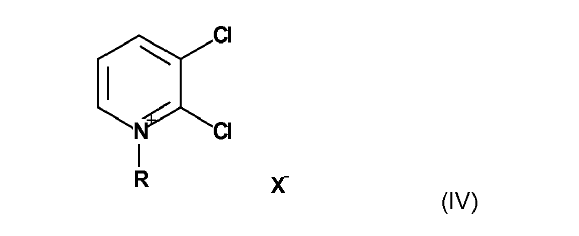 reagieren läßt, aus der durch Erwärmung das 2,3-Dichlorpyridin der Formel (VIII) freigesetzt wird.

2. Verfahren nach Anspruch 1, dadurchgekennzeichnet, dass das Chlorierungsreagenz ein Reagenz ausgewählt aus der Gruppe bestehend aus Phosgen, Oxalylchlorid, Thionylchlorid und Phosporoxytrichlorid, bevorzugt Phosgen ist.

3. Verfahren nach einem der Ansprüche 1 bis 2, **dadurch gekennzeichnet, dass** neben dem Chlorierungsreagenz ein Trialkylamin, insbesonders Trimethylamin, Triethylamin, Triisopropylamin, Tripropylamin oder Tributylmin, zugesetzt wird.

4. Verfahren nach einem der Ansprüche 1 bis 3, **dadurch gekennzeichnet, dass** das Verfahren bei einer Temperatur im Bereich von -25°C bis 120°C durchgeführt wird.

5. Verfahren nach einem der Ansprüche 1 bis 4, **dadurch gekennzeichnet, dass** das Verfahren in einem inerten Lösungsmittel ausgewählt aus der Gruppe bestehend aus Hexan, Methylcyclohexan, Toluol, Xylol, Chlorbenzol, Dichlorbenzol, Trichlorbenzol und Ethylbenzol, durchgeführt wird.

6. Verfahren nach einem der Ansprüche 1 bis 2, **dadurch gekennzeichnet, dass** man das 2,3-Dichlorpryridin der Formel (VIII) aus der 2,3-Dichloryridiniumchlorid-Verbindung der Formel (IV) durch Erwärmung auf eine Temperatur im Bereich zwischen 60°C und 160°C freisetzt.

7. Verfahren nach einem der Ansprüche 1 bis 6, **dadurch gekennzeichnet, dass** man das Vilsmeier-Reagenz der Formel (III) separat oder in situ durch die Reaktion des Chlorierungsreagenz, insbesondere Phosgen, mit einem Dialkylformamid der Formel (IX) 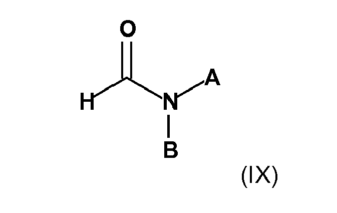 in der A und B die für Formel (III) angegebene Bedeutung besitzen,
herstellt.

8. Verfahren nach Anspruch 7, **dadurch gekennzeichnet, dass** das Dialkylamid der Formel (IX) N, N-Dimethylformamid, N,N- Di-n-butylformamid oder N-Methyl-N-phenylformamid ist.

9. Verfahren nach einem der Ansprüche 1 bis 8, **dadurch gekennzeichnet, dass** R für einen Rest ausgewählt aus der Gruppe Phenyl, Methyl, Ethyl, n-Propyl, oder iso-Propyl, n-Butyl, iso-Butyl und Benzyl steht.

10. Verfahren nach einem der Ansprüche 1 bis 9, **dadurch gekennzeichnet, dass** man pro Mol N-Ethenylchloracetamid der Formel (II) 1 bis 1,6 Mol Vilsmeier-Reagenz der Formel (III) und 1 bis 5 Mol Chlorierungsreagenz einsetzt.

11. Verfahren nach Anspruch 1, **dadurch gekennzeichnet, dass** man das Vilsmeier-Reagenz der Formel (III) herstellt, indem man ein Dialkylformamid der Formel (IX) 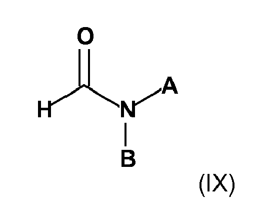 in der A und B die für Formel (III) angegebene Bedeutung besitzen, gemeinsam im Gemisch mit dem Ethenylchloracetamid der Formel (II) parallel zu dem Chlorierungsreagenz in ein vorgelegtes Lösungsmittel eindosiert.
